# EUROPEAN PATENT SPECIFICATION

(11) **EP 3 600 326 B1**
(45) Date of publication and mention of the grant of the patent: **25.01.2023**
(21) Application number: 18718926.1
(22) Date of filing: 29.03.2018
(51) Int. Cl.: A61K 31/506, A61K 45/06, A61P 35/02

(54) **DOSE AND REGIMEN FOR AN HDM2-P53 INTERACTION INHIBITOR IN HEMATOLOGICAL TUMORS**
DOSIS UND DOSIERSCHEMA FÜR EINEN HDM2-P53-WECHSELWIRKUNGSHEMMER BEI HÄMATOLOGISCHEN TUMOREN
DOSE ET RÉGIME POSOLOGIQUE POUR UN INHIBITEUR D'INTERACTION HDM2-P53 DANS DES TUMEURS HÉMATOLOGIQUES

(30) Priority: 31.03.2017 US 201762479397 P
(43) Date of publication of application: 05.02.2020
(73) Proprietor: Novartis AG, 4056 Basel (CH)
(72) Inventor: FERRETTI, Stephane, 4002 Basel (CH); GUERREIRO, Nelson, 4056 Basel (CH); JEAY, Sebastien, 4002 Basel (CH); JULLION, Astrid, 4056 Basel (CH); MEILLE, Christophe, 4056 Basel (CH); WUERTHNER, Jens, 4002 Basel (CH); FABRE, Claire, 4002 Basel (CH)
(74) Representative: Strang, Andrea Josephine
(86) International application number: PCT/IB2018/052187
(87) International publication number: WO 2018/178925

(56) References cited:
- WO-A1-2015/198266
- WO-A1-2017/037579
- WO-A1-2018/092020
- KOJIMA KENSUKE ET AL: "Pharmacological activation of wild-type p53 in the therapy of leukemia", EXPERIMENTAL HEMATOLOGY, ELSEVIER INC, US, vol. 44, no. 9, 18 June 2016 (2016-06-18), pages 791-798, XP029690907, ISSN: 0301-472X, DOI: 10.1016/J.EXPHEM.2016.05.014
- D M Hyman ET AL: "Dose- and Regimen-finding Phase I Study of NVP-HDM201 in Patients With TP53 Wild-type Advanced Tumors (Poster presented at EORTC-NCI-AACR Munich, Germany, 29.11.2016-2.12.2016)", , 2 December 2016 (2016-12-02), XP055486600, Retrieved from the Internet: URL:http://www.poster-submission.com/ena20 16/visitors/eposter/33139 [retrieved on 2018-06-21]
- Anonymous: "Archive History for NCT02143635 - Study to Determine and Evaluate a Safe and Tolerated Dose of HDM201 in Patients With Selected Advanced Tumors That Are TP53wt", , 2 February 2017 (2017-02-02), XP055486683, Retrieved from the Internet: URL:https://clinicaltrials.gov/ct2/history /NCT02143635?V_15=View#StudyPageTop [retrieved on 2018-06-21]
- Anonymous: "Early-Phase Trials of HDM201 Show Promise in Leukemias - ASH Clinical News", , 1 May 2017 (2017-05-01), XP055486592, Retrieved from the Internet: URL:https://www.ashclinicalnews.org/meetin g-news/early-phase-trials-hdm201-show-prom ise-leukemias/ [retrieved on 2018-06-21]
- Tomohiro Kurokawa ET AL: "The Eltrombopag antitumor effect on hepatocellular carcinoma", International journal of oncology, vol. 47, no. 5, 1 November 2015 (2015-11-01), pages 1696-1702, XP055620807, GR ISSN: 1019-6439, DOI: 10.3892/ijo.2015.3180

## Description

### FIELD OF THE INVENTION

The present invention relates the HDM2-p53 interaction inhibitor (S)-5-(5-Chloro-1-methyl-2-oxo-1,2-dihydro-pyridin-3-yl)-6-(4-chloro-phenyl)-2-(2,4-dimethoxy-pyrimidin-5-yl)-1-isopropyl-5,6-dihydro-1H-pyrrolo[3,4-d]imidazol-4-one (HDM201) for use in the treatment of hematological tumors, wherein the drug is administered following an extended low dose regimen as defined in the appended claims.

### BACKGROUND OF THE INVENTION

p53 is induced and activated by a number of potentially tumorigenic processes - including aberrant growth signals, DNA damage, ultraviolet light, and protein kinase inhibitors (Millard M, et al. Curr Pharm Design 2011;17:536-559) - and regulates genes controlling cell growth arrest, DNA repair, apoptosis, and angiogenesis (Bullock AN & Fersht AR. Nat Rev Cancer 2001;1:68-76; Vogelstein B, et al. Nature Education 2010;3(9):6).

Human Double Minute-2 (HDM2) is one of the most important regulators of p53. It binds directly to p53, inhibiting its transactivation, and subsequently directing it towards cytoplasmic degradation (Zhang Y, et al. Nucleic Acids Res 2010;38:6544-6554).

p53 is one of the most frequently inactivated proteins in human cancer, either through direct mutation of the *TP53* gene (found in approximately 50% of all human cancers) (Vogelstein, B et al. Nature 2000;408:307-310) or via suppressive mechanisms such as overexpression of HDM2 (Zhao Y, et al. BioDiscovery 2013;8:4).

Potent and selective inhibitors of the HDM2-p53 interaction (also referred to as HDM2 inhibitors or MDM2 inhibitors), e.g. NVP-HDM201, have been shown to restore p53 function in preclinical cell and in vivo models (Holzer P, et al. Poster presented at AACR 2016, Abstract #4855).

Different dosing regimens were described for HDM2 inhibitors and tested in clinical studies. E.g. US2013/0245089 discloses a method of treating a patient suffering from cancer by administering to the patient 4-{[(2R,3S,4R,5S)-4-(4-Chloro-2-fluoro-phenyl)-3-(3-chloro-2-fluoro-phenyl)-4-cyano-5-(2, 2-dimethyl-propyl)-pyrrolidine-2-carbonyl]-amino}-3-methoxybenzoic acid in an amount of from about 800 to about 3000 mg/day for an administration period of up to about 7 days, on days 1-7, of a 28 days treatment cycle, followed by a rest period of from about 21 to about 23 days.

A paper in Clinical Cancer Research by B. Higgins et al. (May 2014) disclosed a 28-day cycle schedule, where RG7388 is administered once weekly three times followed by 13 days of rest (28 days cycle schedule), or where the drug is administered for 5 consecutive days of a 28 days schedule.

Further dosing regimens for HDM2 inhibitors are disclosed in WO 2015/198266.

KOJIMA KENSUKE ET AL: "Pharmacological activation of wild-type p53 in the therapy of leukemia", EXPERIMENTAL HEMATOLOGY, ELSEVIER INC, US, vol. 44, no. 9, 18 June 2016 (2016-06-18), pages 791-798, XP029690907, ISSN: 0301-472X, DOI: 10.1016/J.EXPHEM.2016.05.014 discusses pharmacological activation of wild-type p53 in the therapy of leukemia

WO 2015/198266 A1 discloses mdm2 inhibitors for use in specific dosing schedules.

WO 2017/037579 A1 discloses a pharmaceutical combination comprising (a) an Mdm2 inhibitor and (b)(i) a MEK inhibitor and/or (b)(ii) Bcl2 inhibitor, particularly for use in the treatment of a cancer.

D M Hyman ET AL: "Dose- and Regimen-finding Phase I Study of NVPHDM201 in Patients With TP53 Wild-type Advanced Tumors discusses a dose- and regimen-finding phase I study of NVP-HDM201 in patents (pts) with TP53 wild-type (wt) advanced tumors.

Anonymous: "Archive History for NCT02143635 - Study to Determine and Evaluate a Safe and Tolerated Dose of HDM201 in Patients With Selected Advanced Tumors That Are TP53wt", 2 February 2017 (2017-02-02), XP055486683 discusses a study to determine and evaluate a safe and tolerated dose of HDM201 in patients with selected advanced tumors that are TP53wt.

The HDM2 inhibitor HDM201, i.e. (S)-5-(5-Chloro-1-methyl-2-oxo-1,2-dihydro-pyridin-3-yl)-6-(4-chloro-phenyl)-2-(2,4-dimethoxy-pyrimidin-5-yl)-1-isopropyl-5,6-dihydro-1H-pyrrolo[3,4-d]imidazol-4-one, and methods how to prepare it were disclosed for example in WO2013/111105.

### SUMMARY OF THE INVENTION

One of the objectives in the development of an HDM2 inhibitor drug is to find a dosing regimen which allows the administration of a dose which ensures efficacy but at the same time reduces the risk of the occurance of adverse events.

It has been surprisingly found that one type of dosing regimen is particularly useful for the treatment of hematological tumors with the HDM2 inhibitor HDM201.

Specifically, the present invention provides the following aspects, advantageous features and specific embodiments, respectively alone or in combination, as listed in the following items:
1. The HDM2-p53 interaction inhibitor drug (S)-5-(5-Chloro-1-methyl-2-oxo-1,2-dihydro-pyridin-3-yl)-6-(4-chloro-phenyl)-2-(2,4-dimethoxy-pyrimidin-5-yl)-1-isopropyl-5,6-dihydro-1H-pyrrolo[3,4-d]imidazol-4-one (HDM201) or a pharmaceutically acceptable non-covalent derivative (including salt, solvate, hydrate, complex, co-crystal) thereof
   for use in the treatment of hematological tumors,
   wherein the drug is administered on each of the first 6 to 8 days of a 28 days treatment cycle,
   wherein the treatment is composed of at least two 28 days treatment cycles, and
   wherein the daily drug dose is from 40 mg to 90 mg.
2. The HDM2-p53 interaction inhibitor drug HDM201, or non-covalent derivative thereof, for use in the treatment of hematological tumors according to item 1, wherein the daily drug dose is from 40 mg to 60 mg.
3. The HDM2-p53 interaction inhibitor drug HDM201, or non-covalent derivative thereof, for use in the treatment of hematological tumors according to item 1, wherein the daily drug dose is from 40 mg to 50 mg.
4. The HDM2-p53 interaction inhibitor drug HDM201, or non-covalent derivative thereof, for use in the treatment of hematological tumors according to item 1, wherein daily drug dose is 45 mg.
5. The HDM2-p53 interaction inhibitor drug HDM201, or non-covalent derivative thereof, for use in the treatment of hematological tumors according to item 1, wherein the drug is administered once daily on each of the first 7 days (first week) of a 28 days (4 weeks) treatment cycle and the daily drug dose is 45 mg.
6. The HDM2-p53 interaction inhibitor drug HDM201, or non-covalent derivative thereof, for use in the treatment of hematological tumors according to any one of items 1 - 5, wherein the drug is present as co-crystal, preferably present as succinic acid co-crystal.
7. The HDM2-p53 interaction inhibitor drug HDM201, or non-covalent derivative thereof, for use in the treatment of hematological tumors according to any one of items 1 - 5, wherein the drug is present as solvate, preferably present as hydrate.
8. The HDM2-p53 interaction inhibitor drug HDM201, or non-covalent derivative thereof, for use in the treatment of hematological tumors according to any one of items 1 - 5, wherein the drug is present as non-covalent derivative, preferably present as non-covalent derivative comprising succinic acid or water, more preferably present as non-covalent derivative comprising succinic acid.
9. The HDM2-p53 interaction inhibitor drug HDM201, or non-covalent derivative thereof, for use in the treatment of hematological tumors according to any one of items 1 - 8, wherein the hematological tumor is a leukemia.
10. The HDM2-p53 interaction inhibitor drug HDM201, or non-covalent derivative thereof, for use in the treatment of hematological tumors according to any one of items 1 - 9, wherein the hematological tumor is selected from acute myeloid leukemia (AML), myelodysplastic syndrome (MDS), and acute lymphoblastic leukemia (ALL).
11. The HDM2-p53 interaction inhibitor drug HDM201, or non-covalent derivative thereof, for use in the treatment of hematological tumors according to any one of items 1-10, wherein the hematological tumor is a TP53 wild-type hematological tumor.
12. The HDM2-p53 interaction inhibitor drug HDM201, or non-covalent derivative thereof, for use in the treatment of hematological tumors according to any one of items 1-11, wherein the hematological tumor is a relapsed/refractory hematological tumor.
13. The HDM2-p53 interaction inhibitor drug HDM201, or non-covalent derivative thereof, for use in the treatment of hematological tumors according to any one of items 1 - 8, wherein the hematological tumor is a relapsed/refractory *TP53* wild-type hematological tumor selected from acute myeloid leukemia (AML), myelodysplastic syndrome (MDS), and acute lymphoblastic leukemia (ALL).

The following items are particularly preferred embodiments of the present invention:
14. The HDM2-p53 interaction inhibitor drug (S)-5-(5-Chloro-1-methyl-2-oxo-1,2-dihydropyridin-3-yl)-6-(4-chloro-phenyl)-2-(2,4-dimethoxy-pyrimidin-5-yl)-1-isopropyl-5,6-dihydro-1H-pyrrolo[3,4-d]imidazol-4-one (HDM201) as succinic acid co-crystal
   for use in the treatment of relapsed/refractory *TP53* wild-type hematological tumors selected from acute myeloid leukemia (AML), myelodysplastic syndrome (MDS), and acute lymphoblastic leukemia (ALL),
   wherein the drug is administered once daily on each of the first 7 days of a 28 days treatment cycle,
   wherein the treatment is composed of at least two 28 days treatment cycles, and wherein the daily drug dose is 45 mg.
15. The HDM2-p53 interaction inhibitor drug (S)-5-(5-Chloro-1-methyl-2-oxo-1,2-dihydropyridin-3-yl)-6-(4-chloro-phenyl)-2-(2,4-dimethoxy-pyrimidin-5-yl)-1-isopropyl-5,6-dihydro-1H-pyrrolo[3,4-d]imidazol-4-one (HDM201) as succinic acid co-crystal
   for use in the treatment of relapsed/refractory *TP53* wild-type acute myeloid leukemia (AML),
   wherein the drug is administered once daily on each of the first 7 days of a 28 days treatment cycle,
   wherein the treatment is composed of at least two 28 days treatment cycles, and wherein the daily drug dose is 45 mg.

The dosing regimens of the present invention as described above provide a highly favorable therapeutic index, low incidence of grade 3/4 thrombocytopenia while achieving therapeutically relevant exposures, p53 pathway activation (GDF-15 upregulation), and clinical activity.

### BRIEF DESCRIPTION OF THE DRAWINGS

In the following the present invention is described in detail with reference to accompanying figures in which:
**Figure 1** illustrates the best percentage change in blast percentage in bone marrow (BM) aspirate in AML patients (patients with available bone marrow aspirate).
*Ongoing treatment; #best percentage change is ≥100; TF: treatment failure; CR: complete response; CRi: morphologic CR with incomplete blood count recovery. Daily doses: Regimen 1A: 250, 350 or 400 mg. Regimen 1B: 150 mg. Regimen 2A: 20, 30 mg. Regimen 2C: 45 mg.
**Figure 2** shows the individual average concentration during first treatment cycle versus dose per regimen for patients with hematological tumors.
Line at 120 ng/mL = 95% tumor regression from human SJSA-1 xenograft rat. Line at 41 ng/mL = Average concentration for tumor stasis derived from TGI PK/PD modelling in human SJSA-1 (osteosarcoma) xenograft rat. Line at 19 ng/mL = Average concentration for tumor stasis derived from TGI PK/PD modelling in human HSAX2655 (liposarcoma) PDX rat. Calculation of average dose level (mg/day):

| Regimen | Daily dose (mg) | No. of administration days | Total dose per cycle (mg) | Cycle duration (days) | Average dose (mg/day) |
|---|---|---|---|---|---|
| 1A | 250 | 1 | 250 | 21 | 11.9 |
| | 350 | 1 | 350 | 21 | 16.7 |
| | 400 | 1 | 400 | 21 | 19 |
| 1B | 150 | 2 | 300 | 28 | 10.7 |
| 2A | 20 | 14 | 280 | 28 | 10 |
| | 30 | 14 | 420 | 28 | 15 |
| 2C | 45 | 7 | 315 | 28 | 11.3 |

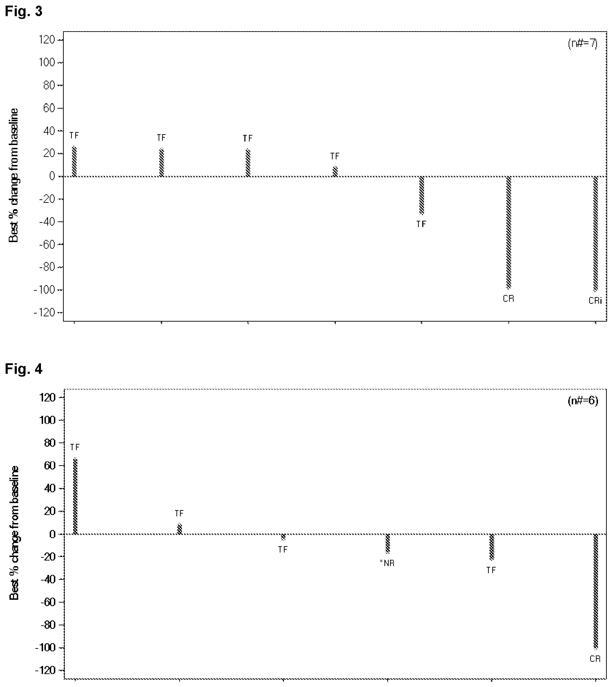

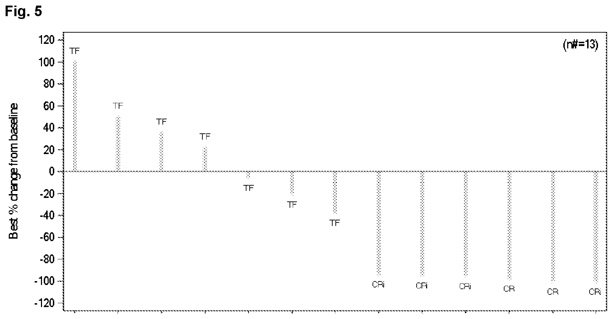

**Figures 3 - 5** illustrates the best percentage change in blast percentage in bone marrow (BM) aspirate in AML patients (cut-off date 15 January 2018).
*Ongoing treatment; #best percentage change is ≥100; TF: treatment failure; CR: complete response; CRi: morphologic CR with incomplete blood count recovery.
Daily doses: **Figure 3**: Regimen 1A at 250 mg. **Figure 4****:** Regimen 1B at 120 mg. **Figure 5****:** Regimen 2C at 45 mg. Dosing regimens 1A, 1B and 2A are reference examples.

### DETAILED DESCRIPTION OF THE INVENTION

Herein after, the present invention is described in further detail and is exemplified.

In one aspect the invention provides:
The HDM2-p53 interaction inhibitor drug (S)-5-(5-Chloro-1-methyl-2-oxo-1,2-dihydropyridin-3-yl)-6-(4-chloro-phenyl)-2-(2,4-dimethoxy-pyrimidin-5-yl)-1-isopropyl-5,6-dihydro-1H-pyrrolo[3,4-d]imidazol-4-one (HDM201) or a pharmaceutically acceptable non-covalent derivative (including salt, solvate, hydrate, complex, co-crystal) thereof
for use in the treatment of hematological tumors,
wherein the drug is administered on each of the first 6 to 8 days, preferably 7 days, of a 28 days treatment cycle,
wherein the treatment is composed of at least two 28 days treatment cycles, and wherein the daily drug dose is from 40 mg to 90 mg, preferably 45 mg.

The term "HDM2-p53 interaction inhibitor" or in short "HDM2 inhibitor" is also referred to as "HDM2i", "Hdm2i", "MDM2 inhibitor", "MDM2i", "Mdm2i", denotes herein any compound inhibiting the HDM-2/p53 or HDM-4/p53 interaction with an IC₅₀ of less than 10 µM, preferably less than 1 µM, preferably in the range of nM, measured by a Time Resolved Fluorescence Energy Transfer (TR-FRET) Assay. The inhibition of p53-Hdm2 and p53-Hdm4 interactions is measured by time resolved fluorescence energy transfer (TR-FRET). Fluorescence energy transfer (or Foerster resonance energy transfer) describes an energy transfer between donor and acceptor 5 fluorescent molecules. For this assay, MDM2 protein (amino acids 2-188) and MDM4 protein (amino acids 2-185), tagged with a C-terminal Biotin moiety, are used in combination with a Europium labeled streptavidin (Perkin Elmer, Inc., Waltham, MA, USA) serving as the donor fluorophore. The p53 derived, Cy5 labeled peptide Cy5- TFSDLWKLL (p53 aa18-26) is the energy acceptor. Upon excitation of the donor 10 molecule at 340nm, binding interaction between MDM2 or MDM4 and the p53 peptide induces energy transfer and enhanced response at the acceptor emission wavelength at 665nm. Disruption of the formation of the p53-MDM2 or p53-MDM4 complex due to an inhibitor molecule binding to the p53 binding site of MDM2 or MDM4 results in increased donor emission at 615nm. The ratiometric FRET assay readout is calculated from the 15 raw data of the two distinct fluorescence signals measured in time resolved mode (countrate 665nm/countrate 615nm x 1000). The assay can be performed according to the following procedure: The test is performed in white 1536w microtiterplates (Greiner Bio-One GmbH, Frickenhausen, Germany) in a total volume of 3.1µl by combining 100nl of compounds diluted in 90% DMSO/10% H2O (3.2% final DMSO concentration) with 2µl Europium 20 labeled streptavidin (final concentration 2.5nM) in reaction buffer (PBS, 125mM NaCl, 0.001% Novexin (consists of carbohydrate polymers (Novexin polymers), designed to increase the solubility and stability of proteins; Novexin Ltd., ambridgeshire, United Kingdom), Gelatin 0.01%, 0.2% Pluronic (block copolymer from ethylenoxide and propyleneoxide, BASF, Ludwigshafen, Germany), 1 mM DTT), followed by the addition of 0.5µl MDM2-Bio or MDM4-Bio diluted in assay buffer (final concentration 10nM). Allow the solution to pre-incubate for 15 minutes at room temperature, followed by addition of 0.5µl Cy5-p53 peptide in assay buffer (final concentration 20nM). Incubate at room temperature for 10 minutes prior to reading the plate. For measurement of samples, an Analyst GT multimode microplate reader (Molecular Devices) with the following settings 30 is used: Dichroic mirror 380nm, Excitation 330nm, Emission Donor 615nm and Emission Acceptor 665nm. IC50 values are calculated by curve fitting using XLfit. If not specified, reagents are purchased from Sigma Chemical Co, St. Louis, MO, USA.

The HDM2 inhibitor in accordance with this invention is HDM201, i.e. (S)-5-(5-Chloro-1-methyl-2-oxo-1,2-dihydro-pyridin-3-yl)-6-(4-chloro-phenyl)-2-(2,4-dimethoxy-pyrimidin-5-yl)-1-isopropyl-5,6-dihydro-1H-pyrrolo[3,4-d]imidazol-4-one.

HDM201 may be present as free molecule or in any other non-covalent derivative, including salt, solvate, hydrate, complex, co-crystal or mixtures thereof. HDM201 may be present as acid derivative. The acid derivative may be a salt formed of HDM201 with the acid, or a HDM201 acid complex, or as HDM201 acid co-crystal. Preferably HDM201 is present as co-crystal. Preferably the acid is succinic acid. Most preferably, HDM201 is present as succinic acid co-crystal. Non-covalent derivatives of HDM201 are described in WO2013/111105.

When referring to a dose amount of HDM201 herein, e.g. in mg (milligram), it is meant to be the amount of HDM201 as free base, in contrast to the salt, solvate, complex, or co-crystal.

The term "hematological tumor" refers herein to a cancer that begins in blood-forming tissue, such as the bone marrow, or in the cells of the immune system. Examples of hematological tumors are leukemia, lymphoma, and multiple myeloma. They are also often referred to as blood cancer.

Preferred hematological tumors of the present invention are leukemias. More preferably, the hematological tumors are selected from acute myeloid leukemia (AML), myelodysplastic syndrome (MDS), and acute lymphoblastic leukemia (ALL). Even more preferably, the hematological tumors are an acute leukemia, preferably selected from acute myeloid leukemia (AML), and acute lymphoblastic leukemia (ALL). Even more preferably, the hematological tumor is AML.

Particularly preferred hematological tumors of the present invention are *TP53* wild-type hematological tumor. More preferably, the *TP53* wild-type hematological tumors of the present invention are *TP53* wild-type leukemias. Even more preferably, the *TP53* wild-type hematological tumors are selected from *TP53* wild-type acute myeloid leukemia (AML), TP53 wild-type myelodysplastic syndrome (MDS), and *TP53* wild-type acute lymphoblastic leukemia (ALL). Even more preferably, the TP53 wild-type hematological tumors are an *TP53* wild-type acute leukemia, preferably selected from TP53 wild-type acute myeloid leukemia (AML), and *TP53* wild-type acute lymphoblastic leukemia (ALL). Even more preferably, the TP53 wild-type hematological tumor is *TP53* wild-type AML.

According to the present invention the drug HDM201 is administered on each of the first 6 to 8 days of a 28 days treatment cycle, preferably on the first seven days (first week) of a 28 days (4 weeks) treatment cycle.

"On the first seven days of a 28 days treatment cycle" means that HDM is administered to the patient on day 1 (d1), d2, d3, d4, d5, d6 and d7 followed by a drug-administration-free period (also referred to as drug holiday period or rest period) from day 8 until day 28. On day 29 the next treatment cycle starts which will be the d1 of this next treatment cycle.

This dosing regimen is also referred to as "1 week on/3 weeks off" or "qd for first week of a 4 week cycle".

Preferably, the drug is administered at approximately the same time each administration day (i.e. d1-d7 of a 28 days cycle). Preferably, the drug is administered once daily (qd) on each administration day. More preferably, the drug is administered in the morning.

Preferably, the drug is administered in the fasted state, i.e. at least 1 hour before or 2 hours after a meal.

Preferably the drug is taken with a glass of water and without chewing the capsules or tablet.

If the patient is assigned to a dose level where multiple capsules/tablets are to be taken, the capsules/tablets should be taken consecutively, within as short an interval as possible, e.g. within 5 min.

Preferably, the drug administration is done by oral delivery, i.e. oral administration, per oral (p.o.).

Preferably the drug is provided in the form of an oral dosage form, more preferably in the form of a solid oral dosage form, e.g. a capsule or a tablet.

When dose ranges are given herein, e.g. "the daily drug dose is from 40 mg to 90 mg", any full mg number of the endpoints and in the between those endpoint shall be meant to be disclosed herewith, e.g. 40 mg, 41 mg, 42 mg, 43 mg, 44 mg, 45 mg, 46 mg, 47 mg, ... 88 mg, 89 mg, 90 mg.

As a further aspect of the present invention there is provided:
A combination of the HDM2-p53 interaction inhibitor drug (S)-5-(5-Chloro-1-methyl-2-oxo-1,2-dihydro-pyridin-3-yl)-6-(4-chloro-phenyl)-2-(2,4-dimethoxy-pyrimidin-5-yl)-1-isopropyl-5,6-dihydro-1H-pyrrolo[3,4-d]imidazol-4-one (HDM201) or a pharmaceutically acceptable non-covalent derivative (including salt, solvate, hydrate, complex, co-crystal) thereof with one or more other therapeutically active agents for use in the treatment of hematological tumors,
wherein the HDM2-p53 interaction inhibitor drug is administered on each of the first 6 to 8 days of a 28 days treatment cycle,
wherein the treatment is composed of at least two 28 days treatment cycles, and wherein the daily drug dose is from 40 mg to 90 mg.

The other active agent may be dosed on the same day(s) as HDM201 or on days on which no HDM201 dose is administered.

The other therapeutically active agent is preferably an anti-cancer agent, more preferably said anti-cancer agent may be selected from:
FLT3 inhibitors (e.g. gilterinib, quizartinib, midostaurin),
BCL2 inhibitors (e.g. navitoclax, venetoclax),
other HDM2 inhibitors (e.g. idasanutlin, AMG232, DS-3032B, ALRN6924/ATSP7041),
hypomethylating agents (HMA) (e.g. Vidaza [azacytidine, 5-azacytidine], Dacogen [decitabine], guadecitabine),
anthracyclines (e.g. idarubicin, daunorubicin, doxorubicin, epirubicin);
anti-CD33 antibodies (e.g. Mylotarg [gemtuzumab], vadastuximab)
and other agents (e.g. AraC [cytarabine, aracytine]).
Preferably, the other therapeutical active agent is selected from midostaurin, azacytidine, cytarabine. Prefered combinations are HDM201 with midostaurin, HDM201 with cytarabine, HDM201 with azacytidine.

### EXAMPLES

### Example 1: Clinical performance of HDM201 administered in dosing regimen 2C

This example provides a summary of the clinical data of the phase 1 trial CHDM201X2101 (data cut-off date of 07-Dec-2016) that demonstrates that the 45 mg single agent HDM201 daily dose with the extended low dose regimen "2C", i.e. 1 week on/3 weeks off regimen, for the for patients with hematological tumors treated according to the (HDM201 given 1 week on/3 weeks off) is the most efficient and safest dose/regimen compared to other extended low dose regimens or other intermittent high dose regimens. That the 45 mg HMD201 dosing regimen 2C if the most efficient dosing regimen has been confirmed by the efficacy data at the cut-off date of 15 Jan 2018.

Herein, data are disclosed from this multicenter, open-label, first-in-human Phase I study of HDM201 in patients with advanced TP53 wild-type (WT) leukemias.

The clinical study design allowed parallel exploration of the safety, tolerability, and clinical activity (efficacy) of two broad dosing strategies for HDM201 during dose escalation: intermittent high dose regimens (Regimen 1A and 1B) and extended low dose regimens (Regimen 2A and 2C). Table 1 summarizes the dosing regimens in each category that were evaluated in patients with hematologic tumors. Dosing regimens 1A, 1B and 2A are reference examples.

**Table 1 HDM201 Dosing regimens and dose levels evaluated in hematologic malignancies**

| | Dosing Regimen | Dose levels (number of patients) | Total number of patients |
|---|---|---|---|
| **Intermittent high dose regimens** | 1A (d1 Q3W) | 250mg (3) | 16 |
| | | 350mg (4) | |
| | | 400mg (8AML; 1ALL) | |
| | 1B (d1,d8 Q4W) | 150mg (6) | 6 |
| **Extended low dose regimens** | 2A (2 week on/2 weeks off) | 20mg (3) | 7 |
| | | 30mg (4) | |
| | 2C (1 week on/3 weeks off) | 45mg (7AML;1 ALL) | 8 |

At the time of data cut-off, a total of 37 patients (35 AML and 2 ALL) have been treated with HDM201 across the 4 dosing regimens evaluated (refer to Table 1). In regimen 2C, 8 patients (7 AML and 1 ALL) have been treated with HDM201 at the single dose level of 45 mg.

Table 2 provides the characteristics of those patients.

**Table 2 Patient characteristics**

| | **Reg 1A (n=16)** | **Reg 1B (n=6)** | **Reg 2A (n=7)** | **Reg 2C (n=8)** |
|---|---|---|---|---|
| Median age, years (range) | 70 (23-81) | 71 (64-83) | 63 (26-72) | 75 (41-81) |
| Male, n (%) | 10 (63) | 1 (17) | 7 (100) | 7 (88) |
| ECOG PS, n (%) | | | | |
| 0 | 2 (13) | 0(0) | 4 (57) | 0(0) |
| 1 | 13 (81) | 5 (83) | 2 (29) | 7 (88) |
| 2 | 1 (6) | 1 (17) | 1 (14) | 1 (13) |

| **Disease history for AML patients** | **n=15** | **n=6** | **n=7** | **n=7** |
|---|---|---|---|---|
| Median prior treatment regimens, n (range) | TBC | TBC | TBC | TBC |
| WHO classification at initial diagnosis, n (%) | | | | |
| AML (BM blasts >30%) | 8 (53) | 6 (100) | 6 (86) | 3 (43) |
| AML with multilineage dysplasia (BM blasts 21-30%) | 6 (40) | 0(0) | 1 (14) | 4 (57) |
| Cytogenetics at initial diagnosis, n (%) | | | | |
| Favorable | 0(0) | 0(0) | 0(0) | 0(0) |
| Intermediate | 3 (20) | 2 (33) | 1 (14) | 2 (29) |
| Unfavorable | 3 (20) | 0(0) | 1 (14) | 2 (29) |
| Unknown | 3 (20) | 2 (33) | 1 (14) | 0(0) |
| Missing | 6 (40) | 2 (33) | 4 (57) | 3 (43) |
| Prior allogeneic stem cell transplant, n (%) | 2 (13) | 1 (17) | 1 (14) | 0(0) |

The patient population is further characterized by the following inclusion criteria:
Patient (male or female) ≥ 18 years of age.

ECOG performance status 0-2.

Relapsed/Refractory AML (both de novo or secondary AML) expect for Acute Promyelocytic Leukemia (APL) with t(15;17), or previously untreated patients who are considered inappropriate candidates for standard induction therapy

In dose escalation only, high and very high risk MDS according to the revised International Prognostic Scoring System (IPSS-R) who have failed prior therapies, such as azacitidine and decitabine (Patients with IPSS-R score of > 4.5).

In dose escalation only, relapsed/Refractory Acute Lymphoblastic Leukemia (B-ALL or T-ALL) including Ph+ ALL, or previously untreated patients who are considered inappropriate candidates for standard induction therapy. Patients with Ph+ ALL who show early markers of relapse in MRD surveillance can be considered for inclusion as long as other therapies such as TKIs are exhausted or cannot be given.

Tumor of the patient is TP53wt characterized by, at a minimum, no mutations in exons 5, 6, 7 and 8, and the p53 status was obtained from a bone-marrow sample, collected no longer than 3 months before signing the main ICF.

### Safety and tolerability profile of regimen 2C

In Regimen 2C the most frequent treatment-related AEs (all grades) were thrombocytopenia and anemia (5 patients each, 62.5%), neutropenia and decreased appetite (4 patients each, 50.0%) febrile neutropenia and nausea (3 patients each, 37.5%). Five patients (62.5%) experienced at least one CTCAE grade 3/4 AE related to study drug. The four most frequent CTCAE grade 3/4 AEs related to study drug were: thrombocytopenia (5 patients, 62.5%), anemia, neutropenia and febrile neutropenia (3 patients each, 37.5%).

One DLT was observed in one patient (12.5%) who had tumor lysis syndrome G4 (Table 3).

This clinical study utilized a Bayesian logistic regression model (BLRM) to support dose escalation and estimate the MTD for HDM201. The BLRM enables incorporation of available prior information and updates the model parameters based upon the DLTs observed in the clinical study at varied doses in evaluable patients. Dose selection for the next cohort is based on EWOC principle which allows only the doses where the probability of exceeding overdose toxicity is less or equal to 25%. The results of the BLRM based on the 1 DLT observed out of 8 evaluable patients in cycle 1 receiving Regimen 2C 45 mg allowed to escalate up to 90 mg.

However, based on the totality of the data observed from all cycles including safety, response rates, PK, PK/PD, the dose of 45 mg for Reg 2C was found most as most preferable for the expansion phase of the clinical study.

**Table 3 Cycle 1 DLTs in hematological tumors**

| | Dosing Regimen | Dose levels (number of evaluable patients) | DLTs |
|---|---|---|---|
| **Intermittent high dose regimens** | 1A (d1 Q3W) | 250mg (3) | 0 |
| | | 350mg (4) | 0 |
| | | 400mg (9) | 4 |
| | Total | N=16 | 4: |
| | | | - Infection G3, GVHd reactivation G3 , Stomatitis G3 |
| | | | -Hypophosphatemia G4 |
| | | | -Subarachnoid hemorrhage (fatal) |
| | | | -Hypophosphatemia G4 |
| | 1B (d1,d8 Q4W) | 150mg (6) | 1 |
| | Total | N=6 | 1: |
| | | | - Acute Kidney Injury G4 |
| **Extended low dose regimens** | 2A (2 weeks on/2 weeks off) | 20mg (3) | 0 |
| | | 30mg (3) | 0 |
| | Total | N=6 | 0 |
| | 2C (1 week on/3 weeks off) | 45mg (8) | 1 |
| | Total | N=8 | 1 - Tumor Lysis Syndrome G4 |

**Table 4 All grades and grade 3/4 adverse events, suspected to be study drug related, by preferred term and regimens - hematological tumors**

| | **HDM201 Regimen 1A subjects** | | **HDM201 Regimen 1B subjects** | | **HDM201 Regimen 2A Subjects** | | **HDM201 Regimen 2C subjects** | | **All subjects** | |
|---|---|---|---|---|---|---|---|---|---|---|
| | **N=16** | | **N=6** | | **N=7** | | **N=8** | | **N=37** | |
| | **All Grades** | **Grade 3/4** | **All Grades** | **Grade 3/4** | **All Grades** | **Grade 3/4** | **All Grades** | **Grade 3/4** | **All Grades** | **Grade 3/4** |
| **MEDDRA Preferred Term** | **n (%)** | **n (%)** | **n (%)** | **n (%)** | **n (%)** | **n (%)** | **n (%)** | **n (%)** | **n (%)** | **n (%)** |
| -Total | 15 (93.8) | 12 (75.0) | 5 (83.3) | 5 (83.3) | 5 (71.4) | 3 (42.9) | 8 (100) | 5 (62.5) | 33 (89.2) | 25 (67.6) |
| Nausea | 10 (62.5) | 0 | 4 (66.7) | 0 | 3 (42.9) | 0 | 3 (37.5) | 0 | 20 (54.1) 0 | |
| Thrombocytop enia/ Platelet Count Decreased | 8 (50.0) | 8 (50.0) | 3 (50.0) | 3 (50.0) | 2 (28.6) | 2 (28.6) | 5 (62.5) | 5 (62.5) | 18 (48.6) | 18 (48.6) |
| Anaemia | 6 (37.5) | 4 (25.0) | 2 (33.3) | 2 (33.3) | 3 (42.9) | 2 (28.6) | 5 (62.5) | 3 (37.5) | 16 (43.2) | 11 (29.7) |
| Neutropenia/ Neutrophil Count Decreased | 6 (37.5) | 6 (37.5) | 1 (16.7) | 1 (16.7) | 1 (14.3) | 1 (14.3) | 4 (50.0) | 3 (37.5) | 12 (32.4) | 11 (29.7) |
| Febrile Neutropenia | 4 (25.0) | 4 (25.0) | 2 (33.3) | 2 (33.3) | 2 (28.6) | 2 (28.6) | 3 (37.5) | 3 (37.5) | 11 (29.7) | 11 (29.7) |
| Decreased Appetite | 4 (25.0) | 0 | 1 (16.7) | 0 | 1 (14.3) | 0 | 4 (50.0) | 0 | 10 (27.0) | 0 |
| Tumour Lysis Syndrome | 7 (43.8) | 7 (43.8) | 0 | 0 | 1 (14.3) | 1 (14.3) | 1 (12.5) | 1 (12.5) | 9 (24.3) | 9 (24.3) |
| Vomiting | 5 (31.3) | 0 | 0 | 0 | 1 (14.3) | 0 | 1 (12.5) | 0 | 7 (18.9) | 0 |
| Diarrhoea | 4 (25.0) | 0 | 0 | 0 | 0 | 0 | 2 (25.0) | 0 | 6 (16.2) | 0 |
| Fatigue | 4 (25.0) | 0 | 0 | 0 | 0 | 0 | 2 (25.0) | 0 | 6 (16.2) | 0 |
| Asthenia | 2 (12.5) | 0 | 1 (16.7) | 0 | 0 | 0 | 2 (25.0) | 0 | 5 (13.5) | 0 |
| Pyrexia | 3 (18.8) | 0 | 0 | 0 | 1 (14.3) | 0 | 0 | 0 | 4 (10.8) | 0 |
| White Blood Cell Count Decreased | 1 (6.3) | 0 | 0 | 0 | 1 (14.3) | 1 (14.3) | 2 (25.0) | 2 (25.0) | 4 (10.8) | 3 (8.1) |
| Amylase Increased | 3 (18.8) | 0 | 0 | 0 | 0 | 0 | 0 | 0 | 3 (8.1) | 0 |
| Lipase Increased | 3 (18.8) | 2 (12.5) | 0 | 0 | 0 | 0 | 0 | 0 | 3 (8.1) | 2 (5.4) |
| Acute Kidney Injury | 1 (6.3) | 0 | 1 (16.7) | 1 (16.7) | 1 (14.3) | 0 | | | 3 (8.1) | 1 (2.7) |
| Blood Creatinine Increased | 1 (6.3) | 0 | 0 | 0 | 1 (14.3) | 0 | 1 (12.5) | 1 (12.5) | 3 (8.1) | 1 (2.7) |
| Blood Phosphorus Increased | 1 (6.3) | 0 | 1 (16.7) | 0 | 0 | 0 | 0 | 0 | 3 (8.1) | 0 |
| Hyperbilirubin aemia | 1 (6.3) | 0 | 1 (16.7) | 0 | 1 (14.3) | 0 | 0 | 0 | 3 (8.1) | 0 |
| Hyperphosph ataemia | 1 (6.3) | 0 | 1 (16.7) | 0 | 0 | 0 | 1 (12.5) | 0 | 3 (8.1) | 0 |
| Abdominal Pain | 2 (12.5) | 0 | 0 | 0 | 0 | 0 | 0 | 0 | 2 (5.4) | 0 |
| Atrial Fibrillation | 2 (12.5) | 0 | 0 | 0 | 0 | 0 | 0 | 0 | 2 (5.4) | 0 |
| Blood Bilirubin Increased | 1 (6.3) | 0 | 1 (16.7) | 0 | 0 | 0 | 0 | 0 | 2 (5.4) | 0 |
| C-Reactive Protein Increased | 1 (6.3) | 0 | 0 | 0 | 1 (14.3) | 1 (14.3) | 0 | 0 | 2 (5.4) | 1 (2.7) |
| Constipation | 0 | 0 | 1 (16.7) | 0 | | | 1 (12.5) | 0 | 2 (5.4) | 0 |
| Dyspepsia | 1 (6.3) | 0 | 0 | 0 | 1 (14.3) | 0 | 0 | 0 | 2 (5.4) | 0 |
| Gamma-Glutamyltransf erase Increased | 2 (12.5) | 1 (6.3) | 0 | 0 | 0 | 0 | 0 | 0 | 2 (5.4) | 1 (2.7) |
| General Physical Health Deterioration | 2 (12.5) | 0 | 0 | 0 | 0 | 0 | 0 | 0 | 2 (5.4) | 0 |
| Gingival Bleeding | 2 (12.5) | 0 | 0 | 0 | 0 | 0 | 0 | 0 | 2 (5.4) | 0 |
| Headache | 1 (6.3) | 0 | 1 (16.7) | 0 | 0 | 0 | 0 | 0 | 2 (5.4) | 0 |
| Hyperuricaemi a | 2 (12.5) | 1 (6.3) | 2 (33.3) | 0 | 1 (14.3) | 0 | | | 2 (5.4) | 1 (2.7) |
| Hypocalcaemi a | 2 (12.5) | 0 | 0 | 0 | 0 | 0 | 0 | 0 | 2 (5.4) | 0 |
| Hypophosphat aemia | 2 (12.5) | 2 (12.5) | 0 | 0 | 0 | 0 | 0 | 0 | 2 (5.4) | 2 (5.4) |
| Hyponatraemi a | 1 (6.3) | 0 | 1 (16.7) | 0 | 0 | 0 | 0 | 0 | 2 (5.4) | 0 |
| Leukopenia | 2 (12.5) | 2 (12.5) | 0 | 0 | 0 | 0 | 0 | 0 | 2 (5.4) | 2 (5.4) |
| Malaise | 1 (6.3) | 0 | 0 | 0 | 1 (14.3) | 0 | 0 | 0 | 2 (5.4) | 0 |
| Mouth Haemorrhage | 2 (12.5) | 0 | 0 | 0 | 0 | 0 | 0 | 0 | 2 (5.4) | 0 |
| Pancytopenia | 0 | 0 | 1 (16.7) | 1 (16.7) | 0 | 0 | 1 (12.5) | 1 (12.5) | 2 (5.4) | 2 (5.4) |
| Stomatitis | 1 (6.3) | 1 (6.3) | 0 | 0 | 0 | 0 | 1 (12.5) | 0 | 2 (5.4) | 1 (2.7) |
| Abdominal Discomfort | 1 (6.3) | 0 | 0 | 0 | 0 | 0 | 0 | 0 | 1 (2.7) | 0 |
| Abdominal Pain Upper | 1 (6.3) | 0 | 0 | 0 | 0 | 0 | 0 | 0 | 1 (2.7) | 0 |
| Arthralgia | 1 (6.3) | 0 | 0 | 0 | 0 | 0 | 0 | 0 | 1 (2.7) | 0 |
| Atypical Pneumonia | 1 (6.3) | 0 | 0 | 0 | 0 | 0 | 0 | 0 | 1 (2.7) | 0 |
| Balanitis Candida | 1 (6.3) | 0 | 0 | 0 | 0 | 0 | 0 | 0 | 1 (2.7) | 0 |
| Blood Alkaline Phosphatase Increased | 1 (6.3) | 0 | 0 | 0 | 0 | 0 | 0 | 0 | 1 (2.7) | 0 |
| Blood Uric Acid Increased | 1 (6.3) | 1 (6.3) | 0 | 0 | 0 | 0 | 0 | 0 | 1 (2.7) | 1 (2.7) |
| Bone Marrow Failure | 0 | 0 | 0 | 0 | 1 (14.3) | 1 (14.3) | 0 | 0 | 1 (2.7) | 1 (2.7) |
| Cardiac Failure | 1 (6.3) | 0 | 0 | 0 | 0 | 0 | 0 | 0 | 1 (2.7) | 0 |
| Cellulitis | 0 | 0 | 1 (16.7) | 1 (16.7) | 0 | 0 | 0 | 0 | 1 (2.7) | 1 (2.7) |
| Chronic Graft Versus Host Disease | 1 (6.3) | 1 (6.3) | 0 | 0 | 0 | 0 | 0 | 0 | 1 (2.7) | 1 (2.7) |
| Conjunctival Haemorrhage | 1 (6.3) | 0 | 0 | 0 | 0 | 0 | 0 | 0 | 1 (2.7) | 0 |
| Cough | 1 (6.3) | 0 | 0 | 0 | 0 | 0 | 0 | 0 | 1 (2.7) | 0 |
| Cystitis Viral | 1 (6.3) | 0 | 0 | 0 | 0 | 0 | 0 | 0 | 1 (2.7) | 0 |
| Cytomegalovir us Infection | 0 | 0 | 0 | 0 | 1 (14.3) | 0 | 0 | 0 | 1 (2.7) | 0 |
| Dyspnoea | 1 (6.3) | 1 (6.3) | 0 | 0 | 0 | 0 | 0 | 0 | 1 (2.7) | 1 (2.7) |
| Epistaxis | 0 | 0 | 0 | 0 | 0 | 0 | 1 (12.5) | 0 | 1 (2.7) | 0 |
| Graft Versus Host Disease | 1 (6.3) | 0 | 0 | 0 | 0 | 0 | 0 | 0 | 1 (2.7) | 0 |
| Haemoglobin Decreased | 1 (6.3) | 0 | 0 | 0 | 0 | 0 | 0 | 0 | 1 (2.7) | 0 |
| Hyperglycaem ia | 1 (6.3) | 0 | 0 | 0 | 0 | 0 | 0 | 0 | 1 (2.7) | 0 |
| Hyperkalaemi a | 0 | 0 | 1 (16.7) | 1 (16.7) | 0 | 0 | 0 | 0 | 1 (2.7) | 1 (2.7) |
| Hypoalbumina emia | 1 (6.3) | 0 | 0 | 0 | 0 | 0 | 0 | 0 | 1 (2.7) | 0 |
| Hypomagnesa emia | 1 (6.3) | 0 | 0 | 0 | 0 | 0 | 0 | 0 | 1 (2.7) | 0 |
| Hypokalaemia | 0 | 0 | 1 (16.7) | 0 | 0 | 0 | 0 | 0 | 1 (2.7) | 0 |
| Hypotension | 1 (6.3) | 0 | 0 | 0 | 0 | 0 | 0 | 0 | 1 (2.7) | 0 |
| Infection | 1 (6.3) | 1 (6.3) | 0 | 0 | 0 | 0 | 0 | 0 | 1 (2.7) | 1 (2.7) |
| Infective Glossitis | 0 | 0 | 0 | 0 | 0 | 0 | 1 (12.5) | 0 | 1 (2.7) | 0 |
| Influenza Like Illness | 1 (6.3) | 0 | 0 | 0 | 0 | 0 | 0 | 0 | 1 (2.7) | 0 |
| Iron Overload | 1 (6.3) | 0 | 0 | 0 | 0 | 0 | 0 | 0 | 1 (2.7) | 0 |
| Lip Oedema | 1 (6.3) | 0 | 0 | 0 | 0 | 0 | 0 | 0 | 1 (2.7) | 0 |
| Macular Oedema | 0 | 0 | 1 (16.7) | 1 (16.7) | 0 | 0 | 0 | 0 | 1 (2.7) | 1 (2.7) |
| Oedema | 1 (6.3) | 0 | 0 | 0 | 0 | 0 | 0 | 0 | 1 (2.7) | 0 |
| Oedema Peripheral | | | 1 (16.7) | 0 | 0 | 0 | 0 | 0 | 1 (2.7) | 0 |
| Oral Disorder | 1 (6.3) | 0 | 0 | 0 | 0 | 0 | 0 | 0 | 1 (2.7) | 0 |
| Pain in Extremity | 1 (6.3) | 0 | 0 | 0 | 0 | 0 | 0 | 0 | 1 (2.7) | 0 |
| Pneumonia | 1 (6.3) | 0 | 0 | 0 | 0 | 0 | 0 | 0 | 1 (2.7) | 0 |
| Pollakiuria | 1 (6.3) | 0 | 0 | 0 | 0 | 0 | 0 | 0 | 1 (2.7) | 0 |
| Rash | 1 (6.3) | 0 | 0 | 0 | 0 | 0 | 0 | 0 | 1 (2.7) | 0 |
| Rash Erythematous | 1 (6.3) | 0 | 0 | 0 | 0 | 0 | 0 | 0 | 1 (2.7) | 0 |
| Rash Papular | 1 (6.3) | 0 | 0 | 0 | 0 | 0 | 0 | 0 | 1 (2.7) | 0 |
| Rectal Haemorrhage | 0 | 0 | 1 (16.7) | 0 | 0 | 0 | 0 | 0 | 1 (2.7) | 0 |
| Renal Failure | 0 | 0 | 0 | 0 | 0 | 0 | 1 (12.5) | 1 (12.5) | 1 (2.7) | 1 (2.7) |
| Skin Exfoliation | 1 (6.3) | 0 | 0 | 0 | 0 | 0 | 0 | 0 | 1 (2.7) | 0 |
| Subarachnoid Haemorrhage | 1 (6.3) | 1 (6.3) | 0 | 0 | 0 | 0 | 0 | 0 | 1 (2.7) | 1 (2.7) |
| Weight Decreased | 1 (6.3) | 0 | 0 | 0 | 0 | 0 | 0 | 0 | 1 (2.7) | 0 |
| Preferred terms are sorted in descending frequency of <all grades> column, as reported in the <All subjects> colum n. | | | | | | | | | | |
| - A subject with multiple occurrences of an AE under one treatment is counted only once in the AE category For that treatment. | | | | | | | | | | |
| - A subject with multiple adverse events is counted only once in the total row. | | | | | | | | | | |
| - Only AEs occurring during treatment or within 30 days of the last study medication are reported. | | | | | | | | | | |

Updated safety data for the dosing regimens 1A (at 250 mg daily dose), 1B (at 120 mg daily dose) and 2C (at 45 mg daily dose) with an cut-off date of 15 January 2018 are provided in the Table 4A.

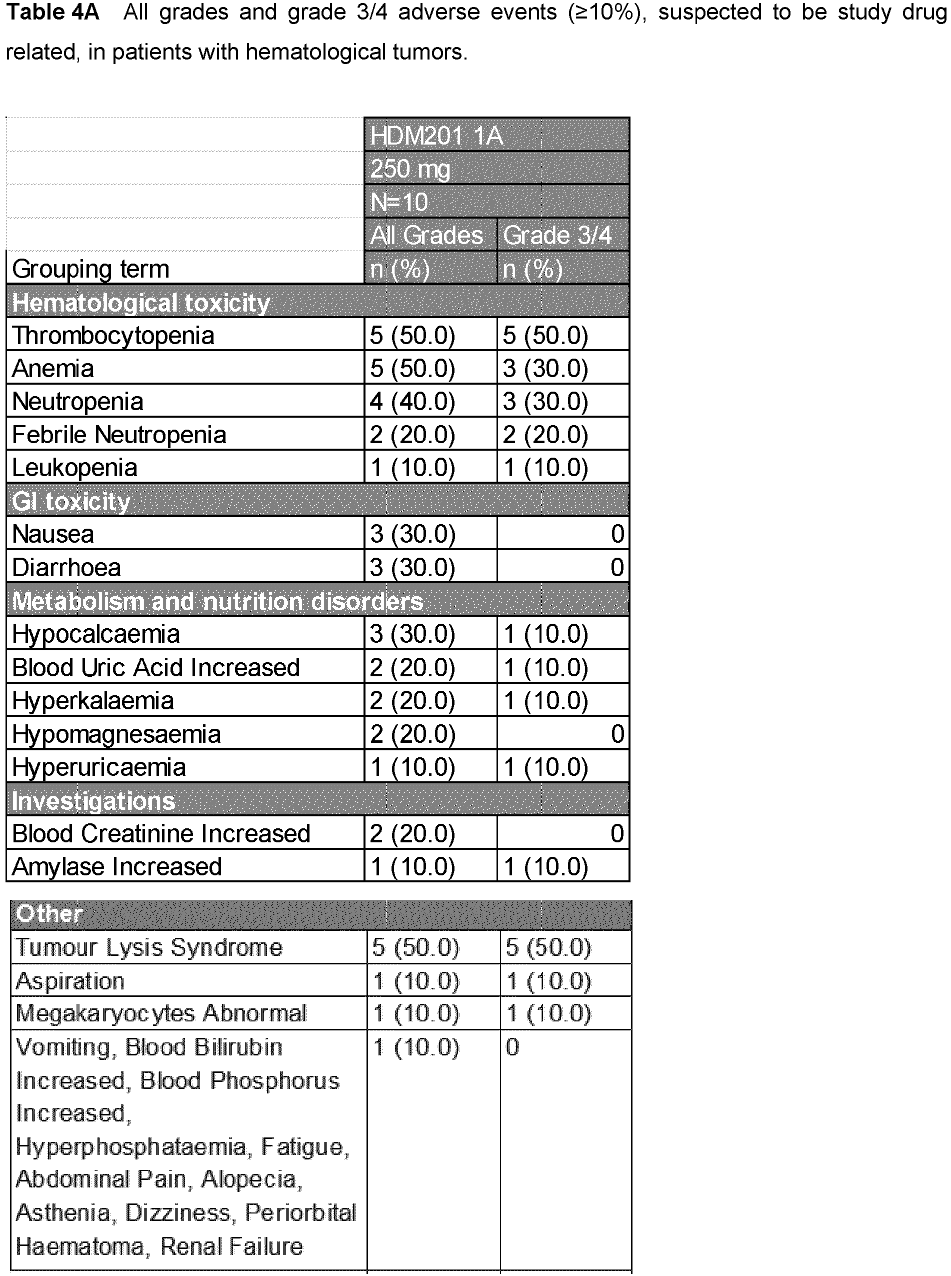

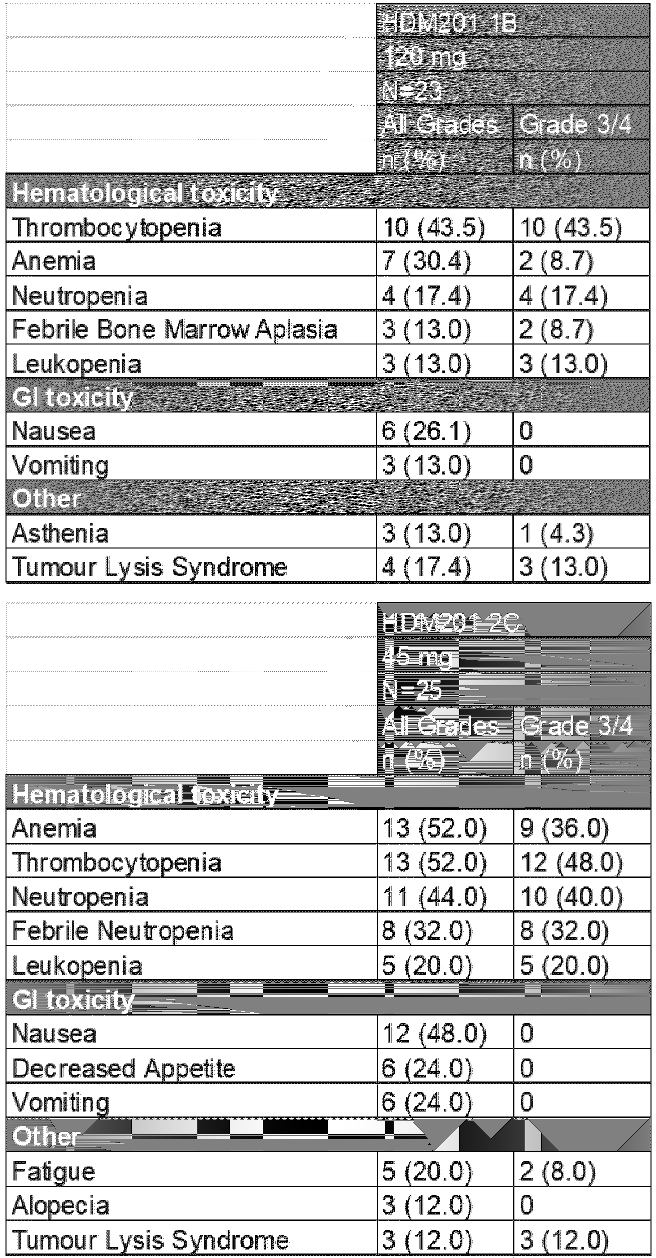

### Anti-tumor activity/efficacy of regimen 2C

Of 8 patients treated at the 45 mg dose in regimen 2C, 7 patients had at least one postbaseline efficacy assessment and the overall response rate was 57.1% (see Table 5). One patient achieved complete response which lasted 152 days and three patients achieved CRi (morphologic CR with incomplete blood count recovery) which lasted 40 days for one of them. For another patient the CRi was ongoing at the time of the data cut off (see Table 6). Compared with the other high dose intermittent (1A and 1B) and the other extended low dose (2A) regimens regimen 2C appears to be clinically most effective.

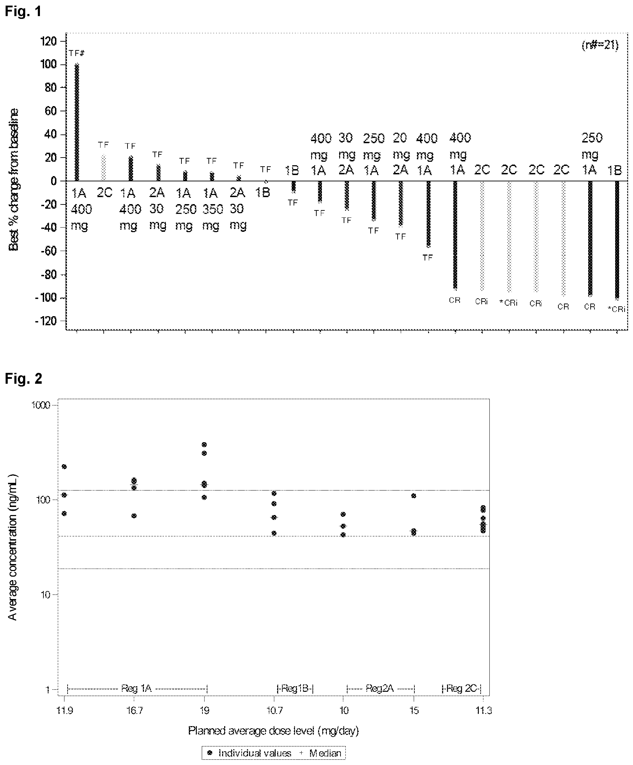

Regimen 2C also shows strong efficacy with respect to the best percentage change in blast percentage in bone marrow (BM) aspirate in AML patients (patients with available bone marrow aspirate), see Figure 1.

Considering that doses up to 30 mg were evaluated in regimen 2A (2 weeks on/2 week off) without evidence of clinically meaningful activity, it appears that a dose much lower than 45 mg in regimen 2C (1 week on/3 weeks off) is less preferred with respect to efficacy.

Although no hematologic toxicity meeting DLT criteria was observed at the 45 mg dose, the patient with CR had a count recovery time of 32 days and the 3 other patients had CR with incomplete count recovery (see Table 6). Given the increased risk of prolonged count recovery times doses much higher than 45mg in regimen 2C appear to be less preferred with respect to tolerability.

**Table 5 Anti-tumor activity**

| **BOR, n (%)** | **Regimen 1A (n=15)** | **Regimen 1B (n=6)** | **Regimen 2A (n=7)** | **Regimen 2C (n=7)** | **Total (N=35 )** |
|---|---|---|---|---|---|
| CR | 2 (13) | 0 (0) | 0 (0) | 1 (14) | 3 (9) |
| CRi | 0 (0) | 1 (17) | 0 (0) | 3 (43) | 4 (11) |
| ORR (CR+CRi+PR) | 2 (13) | 1 (17) | 0(0) | 4 (57) | 7 (20) |

| | | | | | |
|---|---|---|---|---|---|
| BOR, best overall response; CR, complete response; CRi, morphologic CR with incomplete blood count recovery; ORR, overall response rate | | | | | |

**Table 6 Characteristics of AML patients with CR/CRi**

| **Regime n** | **Dose** | **Age (years )** | **Cytogenetics at diagnosis** | **Time to CR/CRi (weeks)** | **Count recovery time from C1D1 (days)** | **Duration of CR/CRi (days)** |
|---|---|---|---|---|---|---|
| **CR patients** | | | | | | |
| Highdose: 1A | 250 mg | 81 | Intermediate | 7 | 48 | 50 |
| Highdose: 1A | 400 mg | 71 | Intermediate | 6 | 40 | 23 |
| Lowdose: 2C | 45 mg | 75 | Missing | 5 | 32 | 152 |

| **CRi patients** | | | | | | |
|---|---|---|---|---|---|---|
| Highdose: 1B | 150 mg | 83 | Intermediate | 11 | NA | 51 |
| Lowdose: 2C | 45 mg | 80 | Missing | 7 | NA | 40 |
| Lowdose: 2C | 45 mg | 70 | Unfavorable | 12 | NA | 1* |
| Lowdose: 2C | 45 mg | 79 | Intermediate | 4 | NA | 1^{†} |

| | | | | | | |
|---|---|---|---|---|---|---|
| *Patient withdrew consent while on CRi ^{†}Patient ongoing at the time of data cut off | | | | | | |

Updated efficacy data for the dosing regimens 1A (at 250 mg daily dose), 1B (at 120 mg daily dose) and 2C (at 45 mg daily dose) for patients with hematological tumors with a cut-off date of 15 January 2018 are provided in the Figures 3 - 5 and Table 7.

**Table 7 Updated anti-tumor activity (cut-off: 15 January 2018)**

| | **Regimen 1A** | **Regimen 1B** | **Regimen 2C** |
|---|---|---|---|
| Patients (n) | 10 | 23 | 25 |
| CR | 1 | 1 | 2 |
| CRi | 1 | 0 | 4 |
| Time to CR/CRi | 24 - 50 days | 30 days | 34 - 49 days |
| Duration of CR/CRi | 50 - 125 days | 1 day (DoR censored) | 1 (DoR censored) - 152 days |
| Mean duration of exposure | 9.1 weeks | 8.7 weeks | 12.1 weeks |

### Clinical PK

Pharmacokinetic data have been evaluated throughout the course of the clinical study. Noncompartmental PK analysis showed a median time to reach maximum plasma concentrations ranging from 2.0 to 5.8 h across the dose range (2 to 350 mg). A preliminary dose proportionality assessment showed approximately dose proportional PK (AUClast and Cmax) over the dose range studied. For the majority of dose cohorts, the inter-patient variability (CV% Geo-mean) for AUClast and Cmax was low to moderate (6 to 58.5%). Furthermore, an integrated analysis of all available HDM201 concentrations was conducted using a population approach. The PK of HDM201 was best described by a 1-compartment PK model with a delayed zero- and first-order absorption process, and a linear clearance. Body weight was identified as a statistically significant covariate on apparent central volume of distribution (Vc/F), in which Vc/F increased with increasing body weight.

To further support the 45 mg for HDM201, compartmental PK modeling was used to estimate the individual average concentration during cycle 1 for patients with hematological tumors treated at 45 mg on regimen 2C (Figure 2). For all patients with measured PK, the estimated average drug concentrations during cycle 1 were above the most conservative average tumor stasis concentration of about 41 ng/mL per cycle determined from PKPD modeling of preclinical data (human SJSA-1 xenograft rat model).

### Example 2: Drug product

The drug product consists of HDM201 succinic acid drug substance filled directly into hard gelatin capsules (HGC), and does not contain any other excipients. The drug product is provided in four dosage strengths: 1 mg, 2.5 mg, 10 mg and 100 mg (based on the weight of the free form), intended for oral use. The 1 mg strength capsule is a "Size 3" yellow HGC, the 2.5 mg strength capsule is a "Size 3" Swedish Orange HGC, the 10 mg strength capsule is a "Size 1" Grey HGC, and the 100 mg is a "Size 0" Swedish Orange HGC. The drug product is packaged in child resistant, induction sealed High Density Polyethylene (HDPE) bottles.

## Claims

1. The HDM2-p53 interaction inhibitor drug (S)-5-(5-Chloro-1-methyl-2-oxo-1,2-dihydropyridin-3-yl)-6-(4-chloro-phenyl)-2-(2,4-dimethoxy-pyrimidin-5-yl)-1-isopropyl-5,6-dihydro-1H-pyrrolo[3,4-d]imidazol-4-one (HDM201) or a pharmaceutically acceptable non-covalent derivative (including salt, solvate, hydrate, complex, co-crystal) thereof
for use in the treatment of hematological tumors,
wherein the drug is administered on each of the first 6 to 8 days of a 28 days treatment cycle,
wherein the treatment is composed of at least two 28 days treatment cycles, and
wherein the daily drug dose is from 40 mg to 90 mg.

2. The HDM2-p53 interaction inhibitor drug HDM201, or non-covalent derivative thereof, for use in the treatment of hematological tumors according to claim 1, wherein the daily drug dose is from 40 mg to 60 mg, for example from 40 mg to 50 mg.

3. The HDM2-p53 interaction inhibitor drug HDM201, or non-covalent derivative thereof, for use in the treatment of hematological tumors according to claim 1, wherein the daily drug dose is 45 mg.

4. The HDM2-p53 interaction inhibitor drug HDM201, or non-covalent derivative thereof, for use in the treatment of hematological tumors according to claim 1, wherein the drug is administered once daily on each of the first 7 days (first week) of a 28 days (4 weeks) treatment cycle and the daily drug dose is 45 mg.

5. The HDM2-p53 interaction inhibitor drug HDM201, or non-covalent derivative thereof, for use in the treatment of hematological tumors according to any one of claims 1 - 4, wherein the drug is present as co-crystal, preferably present as a succinic acid co-crystal.

6. The HDM2-p53 interaction inhibitor drug HDM201, or non-covalent derivative thereof, for use in the treatment of hematological tumors according to any one of claims 1 - 4, wherein the drug is present as a solvate, preferably present as a hydrate.

7. The HDM2-p53 interaction inhibitor drug HDM201, or non-covalent derivative thereof, for use in the treatment of hematological tumors according to any one of claims 1 - 4, wherein the drug is present as a non-covalent derivative, preferably present as a non-covalent derivative comprising succinic acid or water, more preferably present as a non-covalent derivative comprising succinic acid.

8. The HDM2-p53 interaction inhibitor drug HDM201, or non-covalent derivative thereof, for use in the treatment of hematological tumors according to any one of claims 1 - 7, wherein the hematological tumor is a leukemia.

9. The HDM2-p53 interaction inhibitor drug HDM201, or non-covalent derivative thereof, for use in the treatment of hematological tumors according to any one of claims 1 - 8, wherein the hematological tumor is selected from acute myeloid leukemia (AML), myelodysplastic syndrome (MDS), and acute lymphoblastic leukemia (ALL).

10. The HDM2-p53 interaction inhibitor drug HDM201, or non-covalent derivative thereof, for use in the treatment of hematological tumors according to any one of claims 1 - 9, wherein the hematological tumor is a *TP53* wild-type hematological tumor.

11. The HDM2-p53 interaction inhibitor drug HDM201, or non-covalent derivative thereof, for use in the treatment of hematological tumors according to any one of claims 1-10, wherein the hematological tumor is a relapsed/refractory hematological tumor.

12. The HDM2-p53 interaction inhibitor drug HDM201, or non-covalent derivative thereof, for use in the treatment of hematological tumors according to any one of claims 1 - 7, wherein the hematological tumor is a relapsed/refractory *TP53* wild-type hematological tumor selected from acute myeloid leukemia (AML), myelodysplastic syndrome (MDS), and acute lymphoblastic leukemia (ALL).

13. The HDM2-p53 interaction inhibitor drug (S)-5-(5-Chloro-1-methyl-2-oxo-1,2-dihydropyridin-3-yl)-6-(4-chloro-phenyl)-2-(2,4-dimethoxy-pyrimidin-5-yl)-1-isopropyl-5,6-dihydro-1H-pyrrolo[3,4-d]imidazol-4-one (HDM201) for use in the treatment of hematological tumors according to claim 1,
wherein the HDM201 is present as a succinic acid co-crystal,
wherein the hematological tumor is a relapsed/refractory *TP53* wild-type hematological tumor selected from acute myeloid leukemia (AML), myelodysplastic syndrome (MDS), and acute lymphoblastic leukemia (ALL),
wherein the drug is administered on each of the first 7 days of a 28 days treatment cycle, and
wherein the treatment is composed of at least two 28 days treatment cycles, and wherein the daily drug dose is 45 mg.

14. The HDM2-p53 interaction inhibitor drug (S)-5-(5-Chloro-1-methyl-2-oxo-1,2-dihydropyridin-3-yl)-6-(4-chloro-phenyl)-2-(2,4-dimethoxy-pyrimidin-5-yl)-1-isopropyl-5,6-dihydro-1H-pyrrolo[3,4-d]imidazol-4-one (HDM201) for use in the treatment of hematological tumors according to claim 1,
wherein the HDM201 is present as a succinic acid co-crystal,
wherein the hematological tumor is a relapsed/refractory *TP53* wild-type acute myeloid leukemia (AML),
wherein the drug is administered on each of the first 7 days of a 28 days treatment cycle, and
wherein the treatment is composed of at least two 28 days treatment cycles, and wherein the daily drug dose is 45 mg.

## Patentansprüche

1. HDM2-p53-Wechselwirkungsinhibitor-Arzneistoff (S)-5-(5-Chlor-1-methyl-2-oxo-1,2-dihydropyridin-3-yl)-6-(4-chlorphenyl)-2-(2,4-dimethoxypyrimidin-5-yl)-1-isopropyl-5,6-dihydro-1H-pyrrolo[3,4-d]imidazol-4-on (HDM201) oder ein pharmazeutisch unbedenkliches nichtkovalentes Derivat (einschließlich Salz, Solvat, Hydrat, Komplex, Cokristall) davon
zur Verwendung bei der Behandlung von hämatologischen Tumoren,
wobei der Arzneistoff an jedem der ersten 6 bis 8 Tage eines 28-tägigen Behandlungszyklus verabreicht wird,
wobei die Behandlung aus mindestens zwei 28-tägigen Behandlungszyklen besteht und
wobei die tägliche Arzneistoffdosis 40 mg bis 90 mg beträgt.

2. HDM2-p53-Wechselwirkungsinhibitor-Arzneistoff HDM201 oder nichtkovalentes Derivat davon zur Verwendung bei der Behandlung von hämatologischen Tumoren nach Anspruch 1, wobei die tägliche Arzneistoffdosis 40 mg bis 60 mg, zum Beispiel 40 mg bis 50 mg beträgt.

3. HDM2-p53-Wechselwirkungsinhibitor-Arzneistoff HDM201 oder nichtkovalentes Derivat davon zur Verwendung bei der Behandlung von hämatologischen Tumoren nach Anspruch 1, wobei die tägliche Arzneistoffdosis 45 mg beträgt.

4. HDM2-p53-Wechselwirkungsinhibitor-Arzneistoff HDM201 oder nichtkovalentes Derivat davon zur Verwendung bei der Behandlung von hämatologischen Tumoren nach Anspruch 1, wobei der Arzneistoff an jedem der ersten 7 Tage (erste Woche) eines 28-tägigen (4-wöchigen) Behandlungszyklus einmal täglich verabreicht wird und die tägliche Arzneistoffdosis 45 mg beträgt.

5. HDM2-p53-Wechselwirkungsinhibitor-Arzneistoff HDM201 oder nichtkovalentes Derivat davon zur Verwendung bei der Behandlung von hämatologischen Tumoren nach einem der Ansprüche 1-4, wobei der Arzneistoff als Cokristall, vorzugsweise als Bernsteinsäure-Cokristall, vorliegt.

6. HDM2-p53-Wechselwirkungsinhibitor-Arzneistoff HDM201 oder nichtkovalentes Derivat davon zur Verwendung bei der Behandlung von hämatologischen Tumoren nach einem der Ansprüche 1-4, wobei der Arzneistoff als Solvat, vorzugsweise als Hydrat, vorliegt.

7. HDM2-p53-Wechselwirkungsinhibitor-Arzneistoff HDM201 oder nichtkovalentes Derivat davon zur Verwendung bei der Behandlung von hämatologischen Tumoren nach einem der Ansprüche 1-4, wobei der Arzneistoff als nichtkovalentes Derivat, vorzugsweise als nichtkovalentes Derivat, das Bernsteinsäure oder Wasser umfasst, weiter bevorzugt als nichtkovalentes Derivat, das Bernsteinsäure umfasst, vorliegt.

8. HDM2-p53-Wechselwirkungsinhibitor-Arzneistoff HDM201 oder nichtkovalentes Derivat davon zur Verwendung bei der Behandlung von hämatologischen Tumoren nach einem der Ansprüche 1-7, wobei es sich bei dem hämatologischen Tumor um Leukämie handelt.

9. HDM2-p53-Wechselwirkungsinhibitor-Arzneistoff HDM201 oder nichtkovalentes Derivat davon zur Verwendung bei der Behandlung von hämatologischen Tumoren nach einem der Ansprüche 1-8, wobei der hämatologische Tumor aus akuter myeloischer Leukämie (AML), myelodysplastischem Syndrom (MDS) und akuter lymphoblastischer Leukämie (ALL) ausgewählt ist.

10. HDM2-p53-Wechselwirkungsinhibitor-Arzneistoff HDM201 oder nichtkovalentes Derivat davon zur Verwendung bei der Behandlung von hämatologischen Tumoren nach einem der Ansprüche 1-9, wobei es sich bei dem hämatologischen Tumor um einen hämatologischen Tumor mit TP53-Wildtyp handelt.

11. HDM2-p53-Wechselwirkungsinhibitor-Arzneistoff HDM201 oder nichtkovalentes Derivat davon zur Verwendung bei der Behandlung von hämatologischen Tumoren nach einem der Ansprüche 1-10, wobei es sich bei dem hämatologischen Tumor um einen rezidiven/refraktären hämatologischen Tumor handelt.

12. HDM2-p53-Wechselwirkungsinhibitor-Arzneistoff HDM201 oder nichtkovalentes Derivat davon zur Verwendung bei der Behandlung von hämatologischen Tumoren nach einem der Ansprüche 1-7, wobei es sich bei dem hämatologischen Tumor um einen rezidiven/refraktären hämatologischen Tumor mit *TP53*-Wildtyp, der aus akuter myeloischer Leukämie (AML), myelodysplastischem Syndrom (MDS) und akuter lymphoblastischer Leukämie (ALL) ausgewählt ist, handelt.

13. HDM2-p53-Wechselwirkungsinhibitor-Arzneistoff (S)-5-(5-Chlor-1-methyl-2-oxo-1,2-dihydropyridin-3-yl)-6-(4-chlorphenyl)-2-(2,4-dimethoxypyrimidin-5-yl)-1-isopropyl-5,6-dihydro-1H-pyrrolo[3,4-d]imidazol-4-on (HDM201) zur Verwendung bei der Behandlung von hämatologischen Tumoren nach Anspruch 1,
wobei das HDM201 als Bernsteinsäure-Cokristall vorliegt,
wobei es sich bei dem hämatologischen Tumor um einen rezidiven/refraktären hämatologischen Tumor mit *TP53-*Wildtyp, der aus akuter myeloischer Leukämie (AML), myelodysplastischem Syndrom (MDS) und akuter lymphoblastischer Leukämie (ALL) ausgewählt ist, handelt,
wobei der Arzneistoff an jedem der ersten 7 Tage eines 28-tägigen Behandlungszyklus verabreicht wird und
wobei die Behandlung aus mindestens zwei 28-tägigen Behandlungszyklen besteht und wobei die tägliche Arzneistoffdosis 45 mg beträgt.

14. HDM2-p53-Wechselwirkungsinhibitor-Arzneistoff (S)-5-(5-Chlor-1-methyl-2-oxo-1,2-dihydropyridin-3-yl)-6-(4-chlorphenyl)-2-(2,4-dimethoxypyrimidin-5-yl)-1-isopropyl-5,6-dihydro-1H-pyrrolo[3,4-d]imidazol-4-on (HDM201) zur Verwendung bei der Behandlung von hämatologischen Tumoren nach Anspruch 1,
wobei das HDM201 als Bernsteinsäure-Cokristall vorliegt,
wobei es sich bei dem hämatologischen Tumor um rezidive/refraktäre akute myeloische Leukämie (AML) mit TP53-Wildtyp handelt,
wobei der Arzneistoff an jedem der ersten 7 Tage eines 28-tägigen Behandlungszyklus verabreicht wird und
wobei die Behandlung aus mindestens zwei 28-tägigen Behandlungszyklen besteht und
wobei die tägliche Arzneistoffdosis 45 mg beträgt.

## Revendications

1. Médicament inhibiteur d'interaction HDM2-p53 (S)-5-(5-Chloro-1-méthyl-2-oxo-1,2-dihydro-pyridin-3-yl)-6-(4-chloro-phényl)-2-(2,4-diméthoxy-pyrimidin-5-yl)-1-isopropyl-5,6-dihydro-1H-pyrrolo[3,4-d]imidazol-4-one (HDM201) ou dérivé non covalent pharmaceutiquement acceptable (y compris sel, solvate, hydrate, complexe, cocristal) correspondant
pour une utilisation dans le traitement de tumeurs hématologiques,
dans lequel le médicament est administré à chacun des 6 à 8 premiers jours d'un cycle de traitement de 28 jours, dans lequel le traitement est composé d'au moins deux cycles de traitement de 28 jours, et
dans lequel la dose quotidienne de médicament est de 40 mg à 90 mg.

2. Médicament inhibiteur d'interaction HDM2-p53 HDM201, ou dérivé non covalent correspondant, pour une utilisation dans le traitement de tumeurs hématologiques selon la revendication 1, dans lequel la dose quotidienne de médicament est de 40 mg à 60 mg, par exemple de 40 mg à 50 mg.

3. Médicament inhibiteur d'interaction HDM2-p53 HDM201, ou dérivé non covalent correspondant, pour une utilisation dans le traitement de tumeurs hématologiques selon la revendication 1, dans lequel la dose quotidienne de médicament est de 45 mg.

4. Médicament inhibiteur d'interaction HDM2-p53 HDM201, ou dérivé non covalent correspondant, pour une utilisation dans le traitement de tumeurs hématologiques selon la revendication 1, dans lequel le médicament est administré une fois par jour pendant chacun des 7 premiers jours (première semaine) d'un cycle de traitement de 28 jours (4 semaines) et la dose quotidienne de médicament est de 45 mg.

5. Médicament inhibiteur d'interaction HDM2-p53 HDM201, ou dérivé non covalent correspondant, pour une utilisation dans le traitement de tumeurs hématologiques selon l'une quelconque des revendications 1 à 4, dans lequel le médicament est présent sous forme de cocristal, préférablement sous forme de cocristal avec l'acide succinique.

6. Médicament inhibiteur d'interaction HDM2-p53 HDM201, ou dérivé non covalent correspondant, pour une utilisation dans le traitement de tumeurs hématologiques selon l'une quelconque des revendications 1 à 4, dans lequel le médicament est présent en tant que solvate, préférablement présent en tant qu'hydrate.

7. Médicament inhibiteur d'interaction HDM2-p53 HDM201, ou dérivé non covalent correspondant, pour une utilisation dans le traitement de tumeurs hématologiques selon l'une quelconque des revendications 1 à 4, dans lequel le médicament est présent sous la forme d'un dérivé non covalent, préférablement sous la forme d'un dérivé non covalent comprenant de l'acide succinique ou de l'eau, plus préférablement sous la forme d'un dérivé non covalent comprenant de l'acide succinique.

8. Médicament inhibiteur d'interaction HDM2-p53 HDM201, ou dérivé non covalent correspondant, pour une utilisation dans le traitement de tumeurs hématologiques selon l'une quelconque des revendications 1 à 7, la tumeur hématologique étant une leucémie.

9. Médicament inhibiteur d'interaction HDM2-p53 HDM201, ou dérivé non covalent correspondant, pour une utilisation dans le traitement de tumeurs hématologiques selon l'une quelconque des revendications 1 à 8, dans lequel la tumeur hématologique est choisie parmi la leucémie myéloïde aiguë (LMA), le syndrome myélodysplasique (SMD) et la leucémie lymphoblastique aiguë (LLA).

10. Médicament inhibiteur d'interaction HDM2-p53 HDM201, ou dérivé non covalent correspondant, pour une utilisation dans le traitement de tumeurs hématologiques selon l'une quelconque des revendications 1 à 9, dans lequel la tumeur hématologique est une tumeur hématologique de type sauvage *TP53.*

11. Médicament inhibiteur d'interaction HDM2-p53 HDM201, ou dérivé non covalent correspondant, pour une utilisation dans le traitement de tumeurs hématologiques selon l'une quelconque des revendications 1 à 10, dans lequel la tumeur hématologique est une tumeur hématologique récidivante/réfractaire.

12. Médicament inhibiteur d'interaction HDM2-p53 HDM201, ou dérivé non covalent correspondant, pour une utilisation dans le traitement de tumeurs hématologiques selon l'une quelconque des revendications 1 à 7, dans lequel la tumeur hématologique est une tumeur hématologique récidivante/réfractaire de type sauvage *TP53* choisie parmi la leucémie myéloïde aiguë (LMA), le syndrome myélodysplasique (SMD) et la leucémie lymphoblastique aiguë (LLA).

13. Médicament inhibiteur d'interaction HDM2-p53(S)-5-(5-Chloro-1-méthyl-2-oxo-1,2-dihydro-pyridin-3-yl)-6-(4-chloro-phényl)-2-(2,4-diméthoxy-pyrimidin-5-yl)-1-isopropyl-5,6-dihydro-1H-pyrrolo[3,4-d]imidazol-4-one (HDM201) pour une utilisation dans le traitement de tumeurs hématologiques selon la revendication 1,
dans lequel le HDM201 est présent sous la forme d'un cocristal avec l'acide succinique,
dans lequel la tumeur hématologique est une tumeur hématologique récidivante/réfractaire de type sauvage *TP53* choisie parmi la leucémie myéloïde aiguë (LMA), le syndrome myélodysplasique (SMD) et la leucémie lymphoblastique aiguë (LLA),
dans lequel le médicament est administré pendant chacun des 7 premiers jours d'un cycle de traitement de 28 jours, et
dans lequel le traitement est composé d'au moins deux cycles de traitement de 28 jours, et
dans lequel la dose quotidienne du médicament est de 45 mg.

14. Médicament inhibiteur d'interaction HDM2-p53(S)-5-(5-Chloro-1-méthyl-2-oxo-1,2-dihydro-pyridin-3-yl)-6-(4-chloro-phényl)-2-(2,4-diméthoxy-pyrimidin-5-yl)-1-isopropyl-5,6-dihydro-1H-pyrrolo[3,4-d]imidazol-4-one (HDM201) pour une utilisation dans le traitement de tumeurs hématologiques selon la revendication 1,
dans lequel le HDM201 est présent sous la forme d'un cocristal avec l'acide succinique,
dans lequel la tumeur hématologique est une leucémie myéloïde aiguë (LMA) de type sauvage *TP53* récidivante/réfractaire,
dans lequel le médicament est administré pendant chacun des 7 premiers jours d'un cycle de traitement de 28 jours, et
dans lequel le traitement est composé d'au moins deux cycles de traitement de 28 jours, et
dans lequel la dose quotidienne du médicament est de 45 mg.
